# EUROPÄISCHE PATENTSCHRIFT

(11) **EP 0 437 773 B1**
(45) Veröffentlichungstag und Bekanntmachung des Hinweises auf die Patenterteilung: **26.07.1995**
(21) Anmeldenummer: 90124752.8
(22) Anmeldetag: 19.12.1990
(51) Int. Cl.: C12Q 1/60, C12Q 1/26, C12Q 1/46, C12Q 1/00

(54) **Diagnostisches Mittel zum Nachweis von Cholesterin**
Diagnostic means to detect cholesterol
Moyen de diagnose pour détection de cholestérol

(30) Priorität: 17.01.1990 DE 4001155
(43) Veröffentlichungstag der Anmeldung: 24.07.1991
(73) Patentinhaber: BOEHRINGER MANNHEIM GMBH, 68298 Mannheim (DE)
(72) Erfinder: Rothe, Anselm, Dr. rer. nat., W-6943 Birkenau (DE); Eikmeier, Heino, Dr. rer. nat., W-6143 Lorsch (DE)

(56) Entgegenhaltungen:
- EP-A- 0 016 387
- DE-A- 2 937 012

## Beschreibung

Gegenstand der Erfindung ist ein diagnostisches Mittel zum enzymatischen Nachweis von Cholesterin, ein Verfahren zur Herstellung eines Reagenzfilmes zum Nachweis von Cholesterin, die Verwendung von Skleroproteinen oder Skleroprotein-Hydrolysaten zur Verbesserung diagnostischer Mittel zum Cholesterinnachweis und ein Verfahren zur Bestimmung von Cholesterin.

In zunehmendem Maße wird die Bestimmung von Analyten in Blut vom Patienten ohne Konsultation eines Arztes selbst durchgeführt. Als besonders geeignet hat sich hierbei die Verwendung von Teststreifen erwiesen. Voraussetzung für den Erfolg solcher Patientensysteme sind die einwandfreie Funktion der Teststreifen sowie einfache Handhabbarkeit. Von der die Bestimmung ausführenden Person unabhängige, objektive Ergebnisse können praktisch nur mittels eines Meßgerätes erhalten werden.

Bei der Überwachung des Lipidhaushaltes hat sich die Bestimmung der Cholesterinkonzentration durch den Patienten direkt immer mehr durchgesetzt. Dazu werden im allgemeinen diagnostische Mittel verwendet, welche die für den Nachweis erforderlichen Reagenzien in Form eines Films auf einem Teststreifen beinhalten. Die Herstellung eines solchen Reagenzfilms ist beispielsweise in der EP-B-0 016 387 beschrieben.

Wegen der starken Temperaturabhängigkeit biochemischer Reaktionen besteht jedoch eine ausgeprägte Abhängigkeit der meisten Nachweisreaktionen der klinischen Chemie von der Temperatur der Umgebung. Zur Vermeidung temperaturbedingter Verfälschungen der Analysenergebnisse wird daher im allgemeinen bei konstant gehaltener Temperatur gearbeitet, beispielsweise durch einen temperierten Adapter des Meßgerätes. Für den Erfolg solcher Patientensysteme ist jedoch ein möglichst kleines und preiswertes Meßgerät erwünscht. Mit solchen Kleingeräten ist jedoch nur eine sehr grobe, unzureichende Temperierung des Testträgers möglich.

Aufgabe der vorliegenden Erfindung war es daher, diagnostische Mitte für eine weitgehend temperaturunabhängige Bestimmung von Cholesterin bereitzustellen.

Diese Aufgabe wir durch die Erfindung gelöst.

Gegenstand der Erfindung ist ein diagnostisches Mittel zum enzymatischen Nachweis von Cholesterin, welches neben den notwendigen Enzymen ein Skleroprotein oder Skleroproteinhydrolysat enthält. Diagnostische Mittel zum enzymatischen Nachweis von Cholesterin sind bekannt, beispielsweise aus der EP-B-0 016 387. Diese Mittel enthalten bevorzugt ein enzymatisches Redoxsystem zur Oxidation des Cholesterin. Das Ausmaß der Oxidation des Cholesterin kann über eine Farbbildungsreaktion oder elektrochemisch bestimmt werden. Die bevorzugten Hauptbestandteile eines diagnostischen Mittels, das eine Cholesterinoxidase- und cholesterinesterasehaltige Filmmasse enthält, können der EP-B-0 016 387 entnommen werden.

Das erfindungsgemäße diagnostische Mittel enthält neben den üblichen Reagenzien Cholesterinoxdase und Cholesterinesterase ein Skleroprotein oder Skleroproteinhydrolysat. Besonders bevorzugt sind Skleroproteinhydrolystate aus Collagen oder Elastin mit einem Molekulargewicht zwischen ca. 1 000 und 15 000. Besonders bevorzugt ist Crotein C mit einem Molekulargewicht von ca. 10 000.

Eine besonders bevorzugte Ausführungsform des erfindungsgemäßen diagnostischen Mittels enthält einen Filmbildner, beispielsweise Polyvinylpropionat, mineralische Trägerstoffe wie Kieselgur und Titandioxid, einen geeigneten pH-Puffer, Cholesterinoxidase, Cholesterinesterase, Peroxidase und ein redoxaktives Chromogen, beispielsweise Tetramethylbenzidin. Ein Reagenzfilm mit dieser Zusammensetzung wird bevorzugt als Reagenzfilm in der DE-A-3 130 749 oder der EP-A-0 045 476 eingesetzt.

Im Teststreifen ist der Reagenzfilm bevorzugt auf einer Kunststoffolie angebracht. In JP-A- 566 8607 werden Skleroproteine allgemein als eine unter vielen möglichen Stabilisierungsmitteln für Enzyme beschrieben. Die Verwendung von Skleroproteinen dazu, spezifisch eine Cholesterinbestimmung temperaturunabhängig zu machen, wird nicht angesprochen.

Zur Herstellung eines Reagenzfilmes wird das Skleroprotein oder Skleroproteinhydrolysat der Rohfilmmasse, wie sie beispielsweise aus EP-B-0 016 387 bekannt ist, in einer Konzentration von 1 bis 5 Gew.%, bevorzugt 2 bis 3 Gew.% bezogen auf die Gesamtmasse zugesetzt. Anschießend wird die Masse, bevorzugt auf eine Kunststofffolie, ausgestrichen und getrocknet.

Es hat sich herausgestellt, daß die Verwendung von Skleroproteinen oder Skleroproteinhydrolysaten in enzymhaltigen diagnostischen Mittel zum Nachweis von Cholesterin die Enzymstabilität erhöht. Insbesondere wird jedoch durch den Einsatz von Skleroproteinen oder Skleroproteinhydrolysaten eine weitgehende Temperaturunabhängigkeit des Nachweises von Cholesterin in enzymhaltigen diagnostischen Mitteln erreicht. Dies erlaubt mindestens eine Beschränkung auf eine preisgünstige Grobtemperierung im Meßgerät.

Ebenfalls Gegenstand der vorliegenden Erfindung ist ein Verfahren zur Bestimmung von Cholesterin unter Verwendung eines Reagenzfilms, wobei der Reagenzfilm Cholesterinoxidase und -esterase und die oben genannten Skleroproteine oder Skleroproteinhydrolase enthält. Dazu wird eine flüssige Probe wie Blut, Harn, Serum oder Speichel, bevorzugt Serum, mit einem Reagenzfilm in Kontakt gebracht, welcher die für die Bestimmung erforderlichen Reagenzien und die erfindungsgemäßen Zusätze enthält. Durch die Reagenzien wird das Cholesterin freigesetzt und oxidiert und das dabei gebildete Reduktionsmittel über eine Farbreaktion nachgewiesen. Besonders bevorzugt ist hier die Reaktion mit Peroxidase und Tetramethylbenzidin. Das in der Probe enthaltene Cholesterin kann über die entstehende Färbung, deren Intensität proportional zur Cholesterinmenge ist, optisch, jedoch bevorzugt mittels eines Meßgerätes, quantitativ bestimmt werden.

Die Detektion kann auch remissionsphotometrisch, wie in der EP-A-0 075 766 beschrieben, geschehen.

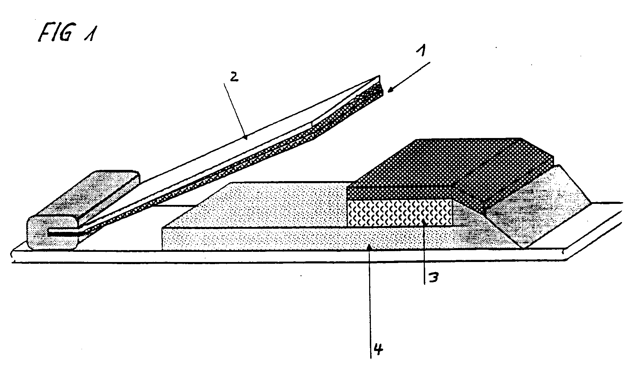

In Fig. 1 ist ein Teststreifen zur Bestimmung von Cholesterin gezeigt, welcher einen erfindungsgemäßen Reagenzfilm 1 auf einer durchsichtigen Trägerfolie 2 enthält. Eine Probe wird auf den Teststreifen auf der rechten Seite aufgegeben, so daß sie ein Erythrozytenabtrennvlies 3 durchfließt und im Transportvlies 4 gesammelt sind. Die Bestimmungsreaktion wird durch Andrücken der Trägerfolie mit dem Reagenzfilm auf das Transportvlies gestartet. Durch die Trägerfolie hindurch wird die Verfärbung des Reagenzfilms beobachtet.

Die folgenden Beispiele erläutern die Erfindung näher:

### Beispiel 1:

### Temperaturabhängigkeit eines Cholesterin-Testes mit Ficoll 70 D-Zusatz.

1) Herstellung eines Reagenzfilmes
Es wird analog EP-A-0016387 eine Filmbeschichtungsmasse mit folgenden Bestandteilen hergestellt:
20 g Polyvinylpropionat-Dispersion 50 % (Propiofan 70 D, BASF)
20 g Kieselgur (Eagle-Pichler-Industries, USA)
3 g Titandioxid (Kronos-Titan, Leverkusen)
10 g Kelzan-Lösung 1%ig (Alginate Industries, Hamburg)
20 g Phosphatpuffer pH 6,4
10 g Wasser
0,5 g Tetramethylbenzidin (Boehringer Mannheim)
1,0 g Dioctylnatriumsulfosuccinat (Sigma-Chemie, Deisenhofen)
0,25 g Gallussäure-Lösung 2,5%ig in Methanol (Merck, Darmstadt)
5 g Tetrahydrofuran p. A. (Merck, Darmstadt)
10 KU Cholesterinoxidase EC 1.1.3.6 (Boehringer Mannheim)
40 KU Cholesterinesterase EC 3.1.1.13 (Boehringer Mannheim)
250 KU Peroxidase EC 1.11.1.7 (Boehringer Mannheim)
2,5 g Ficoll 70 D (Copolymer aus Sucrose und Epichlorhydrin, Firma Serva)
Die Filmmasse wird mit 0,2 mm Naßfilmdicke auf eine durchsichtige, 0,2 mm dicke Polycarbonatfolie (Lonza, Whyl) ausgestrichen und bei 60 °C getrocknet.
2) Aufbau eines Teststreifens
Der Reagenzfilm wird in einen Teststreifen gemäß Figur 1 eingebaut.
3) Messung
Die Messungen geschehen an einem Reflexionsphotometer für Cholesterin-Teststreifen, beispielsweise Reflotron (Boehringer Mannheim GmbH), wie in der EP-B-0 075 766 beschrieben.
Meßwellenlänge 660 nm, variable Temperierung des Teststreifen-Adapters von 30 - 50 °C.

| Meßzyklus: | | |
|---|---|---|
| 1. Phase: | 60 sec | Plasmagewinnung und Temperierung |
| 2. Phase: | 14 sec | Andrücken der Klappe 2 mit dem Reagenzfilm 1 auf das Transportvlies 4, Benetzen, Starten |
| 3. Phase: | 80 sec | Öffnen, Belüften, Reaktion |
| 4. Phase: | 4 sec | Andrücken, Remissionsmessung |

4) Ergebnisse
Die Meßergebnisse sind in Tabelle 1 zusammengestellt. Es ist eine nahezu (reziprok) lineare Abhängigkeit des Meßsignals von der Temperatur erkennbar.

**Tabelle 1**

| Temperaturabhängigkeit eines Cholesterintestes mit Ficoll 70 D-Zusatz | | | | | | |
|---|---|---|---|---|---|---|
| Temperatur des Adapters °C | | 33,0 | 35,5 | 38,0 | 39,5 | 45,5 |
| Serum-Cholesterin (mg/dl) | | | | | | |
| 150 | % Remission | 54,8 | 55,8 | 58,3 | 59,0 | 64,2 |
| | % VK | 0,9 | 2,1 | 2,0 | 3,0 | 1,4 |
| 230 | % Remission | 31,1 | 31,9 | 33,4 | 34,6 | 39,0 |
| | % VK | 3,3 | 1,2 | 2,6 | 2,9 | 2,4 |
| 410 | % Remission | 18,4 | 18,9 | 19,7 | 20,2 | 24,1 |
| | % VK | 2,4 | 2,5 | 3,8 | 4,9 | 3,1 |
| Der Variationskoeffizient (VK) wurde durch 10fach-Messungen pro Temperatur und Konzentration ermittelt. | | | | | | |

### Beispiel 2:

### Temperaturabhängigkeit eines Cholesterin-Testes mit Crotein C-Zusatz.

1) Herstellung eines Reagenzfilmes
Rezeptur der Filmbeschichtungsmasse wie in Beispiel 1 beschrieben, jedoch mit 2,5 g Crotein C (Croda, Nettetal) statt Ficoll 70 D.
2) Aufbau eines Teststreifens
Wie in Beispiel 1.
3) Messung
Wie in Beispiel 1.
4) Ergebnisse
Die Meßergebnisse sind in Tabelle 2 zusammengestellt.
Eine Abhängigkeit des Meßwertes von der Temperatur tritt erst oberhalb von ca. 40 °C auf.
Es zeigt sich keine Beeinflussung im Bereich von 30 - 40 °C.
Das Ergebnis erlaubt eine Beschränkung auf eine preisgünstige Grobtemperierung im Meßgerät.

**Tabelle 2**

| Temperaturabhängigkeit eines Cholesterintestes mit Crotein C-Zusatz | | | | | | |
|---|---|---|---|---|---|---|
| Temperatur des Adapters °C | | 33,0 | 35,5 | 38,0 | 39,5 | 45,5 |
| Serum-Cholesterin (mg/dl) | | | | | | |
| 150 | % Remission | 65,5 | 65,4 | 65,4 | 65,1 | 66,3 |
| | % VK | 0,8 | 0,5 | 0,9 | 0,6 | 0,5 |
| 230 | % Remission | 42,2 | 42,0 | 41,8 | 43,1 | 48,8 |
| | % VK | 1,9 | 2,0 | 2,0 | 1,5 | 2,2 |
| 410 | % Remission | 24,9 | 23,9 | 23,7 | 24,4 | 27,3 |
| | % VK | 1,8 | 1,5 | 1,9 | 3,1 | 1,5 |
| Der VK ist hier besser als bei Verwendung von Ficoll. | | | | | | |

## Patentansprüche

1. Diagnostisches Mittel zum enzymatischen Nachweis von Cholesterin, dadurch gekennzeichnet, daß es neben der Cholesterinoxidase und Cholesterinesterase ein Skleroprotein oder Skleroproteinhydrolysat enthält.

2. Mittel gemäß Anspruch 1 dadurch gekennzeichnet, daß es das Skleroprotein oder Skleroproteinhydrolysat in einer cholesterinoxidase- und cholesterinesterasehaltigen Filmmasse enthält.

3. Verfahren zur Herstellung eines Reagenzfilms zum Nachweis von Cholesterin aus einer Rohfilmmasse, dadurch gekennzeichnet, daß der Rohfilmmasse bei der Herstellung des Reagenzfilms 1-5 Gew.% eines Skleroproteins oder eines Skleroproteinhydrolysats und Cholesterinoxidase und Cholesterinesterase zugesetzt werden.

4. Verwendung von Skleroproteinen oder Skleroproteinhydrolysaten zur Erhöhung der Temperaturunabhängigkeit des Nachweises von Cholesterin mit diagnostischen Mitteln.

5. Verfahren zur Bestimmung von Cholesterin unter Verwendung eines Reagenzfilms, dadurch gekennzeichnet, daß der Reagenzfilm Skleroproteine oder Skleroproteinhydrolysate, Cholesterinoxidase und Cholesterinesterase enthält.

6. Teststreifen zum Nachweis von Cholesterin, welcher einen cholesterinoxidase- und cholesterinesterasehaltigen Reagenzfilm aufweist, dadurch gekennzeichnet, daß der Reagenzfilm ein Skleroprotein oder Skleroproteinhydrolysat enthält.

## Claims

1. Diagnostic agent for the enzymatic determination of cholesterol, characterised in that, besides cholesterol oxidase and cholesterol esterase, it contains a scleroprotein or scleroprotein hydrolysate.

2. Agent according to claim 1, characterised in that it contains the scleroprotein or scleroprotein hydrolysate in a film mass containing cholesterol oxidase and cholesterol esterase.

3. Process for the production of a reagent film for the detection of cholesterol from a crude film mass, characterised in that 1 to 5 wt.% of a scleroprotein or of a scleroprotein hydrolysate and cholesterol oxidase and cholesterol esterase are added to the raw film mass in the production of the reagent film.

4. Use of scleroproteins or scleroprotein hydrolysates for the increasing of the temperature-independence of the detection of cholesterol with diagnostic agents.

5. Process for the determination of cholesterol with use of a reagent film, characterised in that the reagent film contains scleroproteins or scleroprotein hydrolysates, cholesterol oxidase and cholesterol esterase.

6. Test strip for the detection of cholesterol which has a reagent film containing cholesterol oxidase and cholesterol esterase, characterised in that the reagent film contains a scleroprotein or scleroprotein hydrolysate.

## Revendications

1. Agent de diagnostic pour la détection enzymatique du cholestérol, caractérisé en ce qu'il contient, en plus de la cholestérol oxydase et de la cholestérol estérase, une scléroprotéine ou un hydrolysat de scléroprotéine.

2. Agent selon la revendication 1, caractérisé en ce qu'il contient la scléroprotéine ou l'hydrolysat de scléroprotéine dans une masse filmogène contenant de la cholestérol oxydase et de la cholestérol estérase.

3. Procédé de préparation d'une pellicule à réactifs pour la détection du cholestérol, à partir d'une masse filmogène brute, caractérisé en ce que pendant la préparation de la pellicule à réactifs, on ajoute à la masse filmogène brute 1-5 % en poids d'une scléroprotéine ou d'un hydrolysat de scléroprotéine ainsi que de la cholestérol oxydase et de la cholestérol estérase.

4. Utilisation de scléroprotéines ou d'hydrolysats de scléroprotéines pour que la détection du cholestérol à l'aide d'agents de diagnostic soit plus sensible à la température.

5. Procédé de détermination du cholestérol, utilisant une pellicule à réactifs, caractérisé en ce que la pellicule à réactifs contient des scléroprotéines ou des hydrolysats de scléroprotéines, de la cholestérol oxydase et de la cholestérol estérase.

6. Bandes de test pour la détection du cholestérol, qui comprennent une pellicule à réactifs contenant de la cholestérol oxydase et de la cholestérol estérase, caractérisées en ce que la pellicule à réactifs contient une scléroprotéine ou un hydrolysat de scléroprotéine.
